# EUROPÄISCHE PATENTSCHRIFT

(11) **EP 1 035 920 B1**
(45) Veröffentlichungstag und Bekanntmachung des Hinweises auf die Patenterteilung: **24.07.2002**
(21) Anmeldenummer: 98965748.1
(22) Anmeldetag: 03.12.1998
(51) Int. Cl.: B01L 3/00

(54) **KAPILLARAKTIVES TESTELEMENT MIT TRÄGER UND ABDECKUNG**
CAPILLARY ACTIVE TEST ELEMENT HAVING SUPPORT AND COVERING
ELEMENT D'ANALYSE A ACTION CAPILLAIRE AVEC SUPPORT ET REVETEMENT

(30) Priorität: 04.12.1997 DE 19753850
(43) Veröffentlichungstag der Anmeldung: 20.09.2000
(73) Patentinhaber: Roche Diagnostics GmbH, 68298 Mannheim (DE)
(72) Erfinder: ZIMMER, Volker, D-69221 Dossenheim (DE)
(86) Internationale Anmeldenummer: EP9807854
(87) Internationale Veröffentlichungsnummer: WO9929428

(56) Entgegenhaltungen:
- EP-A- 0 010 456
- EP-A- 0 852 336
- WO-A-94/22011
- WO-A-96/28715
- US-A- 4 588 624
- US-A- 5 192 502

## Beschreibung

Die Erfindung betrifft eine Vorrichtung zur Probennahme von flüssigen Proben für analytische Elemente, bei denen die Probe in einem kapillaraktiven Kanal von der Probenaufgabeöffnung zum Bestimmungsort der Probe im analytischen Element transportiert wird und bei denen der kapillaraktive Kanal im wesentlichen von einem Träger, einer Abdeckung und gegebenenfalls einer zwischen Abdeckung und Träger liegenden Zwischenschicht gebildet wird. Außerdem betrifft die Erfindung ein Verfahren zur Aufnahme einer flüssigen Probe in ein analytisches Element mit Hilfe der besagten Vorrichtung.

Zur qualitativen oder quantitativen analytischen Bestimmung von Bestandteilen von Körperflüssigkeiten, insbesondere von Blut, werden oft sogenannte trägergebundene Tests verwendet. Bei diesen sind Reagenzien in entsprechenden Schichten eines festen Trägers eingebettet, der mit der Probe in Kontakt gebracht wird. Die Reaktion von flüssiger Probe und Reagenzien führt bei Anwesenheit eines Zielanalyten zu einem nachweisbaren Signal, insbesondere einem Farbumschlag, weicher visuell oder mit Hilfe eines Geräts, meist reflexionsphotometrisch, ausgewertet werden kann.

Testelemente oder Testträger sind häufig als Teststreifen ausgebildet, die im wesentlichen aus einer länglichen Tragschicht aus Kunststoffmaterial und darauf angebrachten Nachweisschichten als Testfeldern bestehen. Es sind jedoch auch Testträger bekannt, die als quadratische oder rechteckige Plättchen gestaltet sind.

Visuell oder reflexionsphotometrisch auszuwertende Testelemente für die klinische Diagnostik sind wie auch elektrochemische Sensoren und Biosensoren häufig so aufgebaut, daß die Probenauftragszone und die Detektionszone in einer vertikalen Achse übereinander angeordnet liegen. Diese Konstruktionsweise birgt eine Reihe von Problemen. Wenn der probenbeladene Teststreifen zur Vermessung in ein Gerät, beispielsweise ein Reflexionsphotometer, eingebracht werden muß, kann potentiell infektiöses Probenmaterial mit Geräteteilen in Berührung kommen und diese gegebenenfalls kontaminieren. Desweiteren ist, vor allem in den Fällen, in denen die Teststreifen von ungeschulten Personen benutzt werden, beispielsweise bei der Blutzuckerselbstkontrolle von Diabetikern, eine Volumendosierung nur schwer zu realisieren.

Seit kurzem sind Testelemente erhältlich, die einen kapillaren Kanal oder Spalt bereitstellen, mit Hilfe dessen sich zumindest ein Teil der beschriebenen Probleme lösen lassen.

EP-B-0 034 049 beschäftigt sich mit einem Testelement, bei dem die Probe auf eine zentrale Probenaufgabestelle, beispielsweise eine Öffnung in einer Abdeckung, aufgegeben wird und mittels Kapillarkraft an mehrere räumlich von der Probenaufgabestelle getrennte Detektionszonen transportiert wird. Bemerkenswert daran ist, daß für die Geometrie der Probenaufgabeöffnung eine besondere Gestaltung, die auch in EP-B-0 010 456 beschrieben wird, als besonders bevorzugt herausgestellt wird. Eine in der Aufsicht regelmäßig-hexagonale Form der Probenzutrittsöffnung soll der Zentrierung eines Probenflüssigkeitstropfens in die Öffnung dienen. Dadurch soll das Eindringen der Probe in den kapillaraktiven Kanal, der rechtwinklig zur Probenaufgabeöffnung verläuft, erleichtert werden.

Während bei den beschriebenen Kapillarspalt-Testelementen die Probenaufnahme in das Testelement durch eine Öffnung geschieht, die senkrecht auf dem Kapillarspalt steht, wird bei anderen Konzepten die Probenflüssigkeit parallel zur Ausbreitungsrichtung direkt in den Kapillarspalt eingebracht. Am einfachsten geschieht dies dadurch daß das Testelement eine Kante aufweist, in der der Kapillarspalt endet und mit der eine Probenflüssigkeit direkt in Kontakt gebracht und von dem zum kapillaren Flüssigkeitstransport befähigten Kanal aufgenommen wird.

Bei den letztgenannten Testelementen tritt häufig das Problem auf, daß Flüssigkeitstropfen, die an die Probenaufgabeöffnung des Kapillarspalts gebracht werden, nicht in den Spalt einzudringen vermögen. Dieses Phänomen kann unterschiedliche Ursachen haben. Es ist denkbar, daß bei der Herstellung solcher Testelemente die Öffnung fertigungsbedingt nicht die Dimensionen besitzt, die für den Einlaß eines Probentropfens in den Kapillarkanal erforderlich sind, beispielsweise weil die Öffnung beim Ablängen, Schneiden oder Ausstanzen des Testelements verunreinigt oder gequetscht wurde. Eine andere Ursache könnte darin zu sehen sein, daß durch die Hydrophobie der verwendeten Materialien, die häufig für die Fertigung der angesprochenen Testelemente eingesetzt werden, wie beispielsweise hydrophobe Kunststoffe, ein Eindringen der Probe in den Kapillarspalt erschwert, verzögert oder unmöglich gemacht wird. Ein Flüssigkeitstropfen dringt beispielsweise schon dann nicht oder nur sehr langsam in das Innere eines kapillaren Kanals ein, wenn zwar dessen innere Oberflächen hydrophil ausgestattet sind, die Schneidekante aber aufgrund der verwendeten Materialien hydrophob ist.

Die Aufgabe der vorliegenden Erfindung bestand darin, die Nachteile des Standes der Technik zu beseitigen.

Dies wird durch den Gegenstand der Erfindung, wie er in den Patentansprüchen charakterisiert ist, erreicht.

Gegenstand der Erfindung ist eine Vorrichtung zur Probennahme von flüssigen Proben für analytische Elemente, bei der die Probe in einem kapillaraktiven Kanal von der Probenaufgabeöffnung zum Bestimmungsort der Probe im analytischen Element transportiert wird und bei der der kapillaraktive Kanal im wesentlichen von einem Träger, einer Abdeckung und gegebenenfalls einer zwischen Abdeckung und Träger liegenden Zwischenschicht gebildet wird, dadurch gekennzeichnet, daß sich eine Aussparung (5) in Form eines teilweisen Einschnitts in einer den zum kapillaren Flüssigkeitstransport befähigten Kanal (3) bildenden Fläche an der die Probenaufgabeöffnung (4) enthaltenden Seitenfläche der Vorrichtung befindet, wobei der Einschnitt an der Seitenfläche der Vorrichtung beginnt und vom Probennahmeort in Richtung des kapillaraktiven Kanals (3) weist, so daß die der Aussparung (5) gegenüberliegende Fläche frei liegt.

Besonders bevorzugt enthält die erfindungsgemäße Vorrichtung eine solche Aussparung. Jedoch können auch Ausgestaltungsformen realisiert werden, bei denen mehrere, zumindest zwei Aussparungen nebeneinander in einer Fläche vorhanden sind oder sich versetzt auf gegenüberliegenden Flächen befinden. Die Form der Aussparungen unterliegt keinen Beschränkungen. Möglich sind also drei- oder vieleckige, sowie runde oder elliptische Formen. Unregelmäßige Formen sind ebenso nicht ausgeschlossen.

Die Aussparung in einer den kapillaren Kanal bildenden Fläche an der die Probenaufgabeöffnung enthaltenden Seitenfläche, dient dazu, sicherzustellen, daß die Probenflüssigkeit in den kapillaren Kanal eintreten kann. Dies wird dadurch erreicht, daß der Probentropfen an der durch die Aussparung enthaltenden Seitenfläche der Vorrichtung, die der Probenaufgabeöffnung am nächsten liegt, direkt auf eine der Flächen aufgetragen werden kann, die in ihrer Verlängerung die innere Oberfläche der Kapillare bilden. Durch geeignete Wahl der Geometrie und Dimensionen der Aussparung wird erreicht, daß der Flüssigkeitstropfen unabhängig von der genauen Position der Dosierung mit sehr hoher Wahrscheinlichkeit mit der kapillaraktiven Zone in Kontakt kommt und bereitwillig in das Innere der Kapillare eingesaugt wird. Beispielsweise ist die Größe der frei liegenden Fläche so zu wählen, daß ein auf sie aufgebrachter Flüssigkeitstropfen zumindest an einer Stelle mit der kapillaraktiven Zone in Kontakt kommt. Beispielsweise ist eine Dimension der Aussparung. z. B. deren Breite, so zu wählen, daß der Durchmesser des Flüssigkeitstropfens geringfügig größer ist als die gewählte Dimension der Aussparung. Für eine Tropfen von 3 µl hat sich eine Breite der Aussparung von 1 mm als geeignet herausgestellt. Besonders bevorzugt wird das Einsaugen des Probentropfens in den kapillaren Kanal dadurch erreicht, daß die durch die Aussparung frei liegende Fläche hydrophiliert ist und zumindest in Richtung des kapillaren Transportkanals direkt an eine kapillaraktive Zone grenzt.

Hydrophile Oberflächen sind in diesem Zusammenhang wasseranziehende Flächen. Wäßrige Proben, darunter auch Blut, spreiten auf solchen Oberflächen gut. Solche Flächen sind unter anderem dadurch charakterisiert, daß an der Grenzfläche ein Wassertropfen auf ihnen einen spitzen Rand- oder Kontaktwinkel ausbildet. Im Gegensatz dazu wird auf hydrophoben, das heißt wasserabweisenden Oberflächen, an der Grenzfläche zwischen Wassertropfen und Oberfläche ein stumpfer Randwinkel ausgebildet.

Der Randwinkel als Resultat der Oberflächenspannungen der Prüfflüsigkeit und der zu untersuchenden Oberfläche ist als Maß für die Hydrophilie einer Oberfläche geeignet. Wasser hat beispielsweise eine Oberflächenspannung von 72 mN/m. Liegt der Wert der Oberflächenspannung der betrachteten Fläche weit, d. h. mehr als 20 mN/M, unter diesem Wert, so ist die Benetzung schlecht und der resultierende Randwinkel ist stumpf. Eine solche Fläche wird als hydrophob bezeichnet. Nähert sich die Oberflächenspannung dem Wert, der für Wasser gefunden wird, so ist die Benetzung gut und der Randwinkel wird spitz. Wird die Oberflächenspannung dagegen gleich oder größer dem für Wasser gefundenen Wert, so zerläuft der Tropfen und es findet Totalspreitung der Flüssigkeit statt. Ein Randwinkel ist dann nicht mehr zu messen. Flächen, die mit Wassertropfen einen spitzen Randwinkel bilden oder bei denen Totalspreitung eines Wassertropfens beobachtet wird, werden als hydrophil bezeichnet.

Die Bereitschaft einer Kapillare, eine Flüssigkeit aufzusaugen, geht mit der Benetzbarkeit der Kanaloberfläche mit der Flüssigkeit einher. Für wäßrige Proben bedeutet dies, daß eine Kapillare aus einem Material gefertigt werden sollte, dessen Oberflächenspannung nahe an 72 mN/m heranreicht oder diesen Wert übertrifft.

Ausreichend hydrophile Materialien zum Aufbau einer Kapillare, die schnell wäßrige Proben aufsaugt, sind beispielsweise Glas, Metall oder Keramik. Für den Einsatz in Testträgern sind diese Materialien jedoch ungeeignet, da sie einige gravierende Nachteile aufweisen, beispielsweise Bruchgefahr bei Glas oder Keramik, oder Veränderung der Oberflächeneigenschaften mit der Zeit bei zahlreichen Metallen. Üblicherweise werden deshalb zur Fertigung von Testelementen Kunstoffolien oder -formteile eingesetzt. Die verwendeten Kunststoffe übertreffen dabei in der Regel kaum eine Oberflächenspannung von 45 mN/m. Selbst mit den, relativ betrachtet, hydrophilsten Kunststoffen wie beipielsweise Polymethylmethacrylat (PMMA) oder Polyamid (PA) lassen sich - wenn überhaupt - nur sehr langsam saugende Kapillaren aufbauen. Kapillaren aus hydrophoben Kunststoffen wie beispielsweise Polystyrol (PS), Polypropylen (PP) oder Polyethylen (PE) saugen im wesentlichen keine wäßrigen Proben. Hieraus ergibt sich die Notwendigkeit, Kunststoffe für die Verwendung als Konstruktionsmaterial für Testelemente mit kapillaraktiven Kanälen hydrophil auszustatten, das heißt zu hydrophilieren.

In einer bevorzugten Ausführungsform des erfindungsgemäßen analytischen Testelements ist zumindest eine, besser jedoch zwei, ganz besonders bevorzugt zwei sich gegenüberliegende Flächen der die innere Oberfläche des zum kapillaren Flüssigkeitstransports befähigten Kanals bildenden Flächen, hydrophiliert. Ganz bevorzugt ist zumindest die der Aussparung gegenüberliegende, freiliegende Fläche hydrophiliert. Wird mehr als eine Fläche hydrophiliert, so können die Flächen entweder mit der gleichen oder mit unterschiedlichen Methoden hydrophil gemacht werden. Die Hydrophilierung ist vor allem dann notwendig, wenn die Materialien, die den kapillaraktiven Kanal bilden, insbesondere der Träger, selbst hydrophob oder nur sehr wenig hydrophil sind, beispielsweise weil sie aus unpolaren Kunststoffen bestehen. Unpolare Kunststoffe, wie zum Beispiel Polystyrol (PS), Polyethylen (PE), Polyethylenterephthalat (PET) oder Polyvinylchlorid (PVC), sind von Vorteil als Trägermaterialien, weil sie die zu untersuchenden Flüssigkeiten nicht absorbieren und damit das Probenvolumen effektiv von der Nachweisschicht genutzt werden kann. Durch die Hydrophilierung der Oberfläche des Kapillarkanals wird erreicht, daß eine polare, bevorzugt wäßrige Probenflüssigkeit bereitwillig in den kapillaren Kanal eintritt und dort rasch zum Bestimmungsort der Probe transportiert wird. Unter Bestimmungsort wird in diesem Zusammenhang derjenige Ort oder diejenige Zone verstanden, an den bzw. zu der die Probe im analytischen Element transportiert werden soll, um das gewünschte Ergebnis zu erzielen. Ist beispielsweise das analytische Element ein optisch oder photometrisch auszuwertender Testträger, so ist der Bestimmungsort der Probe die Nachweiszone des Testträgers, in der eine Reaktion unter Farbänderung beobachtbar ist. Für den Fall, daß das analytische Element ein elektrochemischer Sensor ist, ist unter dem Bestimmungsort der Probe beispielsweise eine im Sensor integrierte Elektrode, die zu der gewünschten elektrochemischen Nachweisreaktion fähig ist, zu verstehen. Dient das analytische Element selbst nicht zum Nachweis eines Analyten in einer Probe sondern beispielsweise lediglich der Volumendosierung oder der Aufnahme einer bestimmten Menge an Probenmaterial, kann der Bestimmungsort der Probe einfach eine Markierung auf dem analytischen Element sein, bis zu der der Kapillarspalt gefüllt werden muß, um beispielsweise ein bestimmtes Probemindestvolumen abzumessen.

Idealerweise wird die Hydrophilierung der Oberfläche des kapillaren Kanals dadurch erreicht, daß zu seiner Fertigung ein hydrophiles Material eingesetzt wird, das jedoch die Probenflüssigkeit selbst nicht oder nicht wesentlich aufzusaugen vermag. Wo dies nicht möglich ist, kann die Hydrophilierung einer hydrophoben oder nur sehr wenig hydrophilen Oberfläche durch geeignete Beschichtung mit einer stabilen, gegenüber dem Probenmaterial inerten, hydrophilen Schicht erreicht werden, beispielsweise durch kovalente Bindung von photoreaktiv ausgerüsteten, hydrophilen Polymeren auf eine Kunststoffoberfläche, durch Aufbringen netzmittelhaltiger Schichten oder durch Beschichtung von Oberflächen mit Nanokompositen mittels Sol-Gel-Technologie. Darüberhinaus ist es möglich, durch thermische, physikalische oder chemische Behandlung der Oberfläche eine gesteigerte Hydrophilie zu erzielen.

Ganz besonders bevorzugt wird die Hydrophilierung durch die Verwendung von dünnen Schichten oxidierten Aluminiums erreicht. Diese Schichten werden entweder direkt auf die gewünschten Bauteile des Testelements aufgebracht, beispielsweise durch Vakuumbedampfen der Werkstücke mit metallischem Aluminium und anschließende Oxidation des Metalls, oder in Form von Metallfolien oder metallbeschichteten Kunststoffolien für den Testträgeraufbau verwendet, die ebenfalls zur Erzielung der erwünscheten Hydrophilie oxidiert werden müssen. Metallschichtdicken von 1 bis 500 nm sind dabei ausreichend. Die Metallschicht wird anschließend zu Bildung der oxidierten Form oxidiert, wobei sich neben der elektrochemischen, anodischen Oxidation vor allem die Oxidation in Gegenwart von Wasserdampf oder durch Kochen in Wasser als besonders geeignete Methoden herausgestellt haben. Die so erzielten Oxidschichten sind je nach Methode zwischen 0,1 und 500 nm, bevorzugt zwischen 10 und 100 nm dick. Größere Schichtdicken sowohl der Metallschicht als auch der Oxidschicht sind zwar prinzipiell praktisch realisierbar, zeigen aber keine weiteren vorteilhaften Wirkungen.

Ein zweiter Gegenstand der Erfindung ist ein Verfahren zur Aufnahme einer flüssigen Probe, insbesondere einer Körperflüssigkeit wie Blut, Plasma, Serum, Urin, Speichel, Schweiß etc., mit Hilfe einer erfindungsgemäßen Vorrichtung. Dabei wird zunächst die flüssige Probe an der durch die Aussparung unterbrochene Kante der Probenaufgabeöffnung mit der Vorrichtung kontaktiert. Durch Kapillarkräfte in den zum kapillaren Flüssigkeitstransport befähigten Kanal wird die Probenflüssigkeit in das Innere der Vorrichtung transportiert, so daß sie ihren Bestimmungsort erreichen kann.

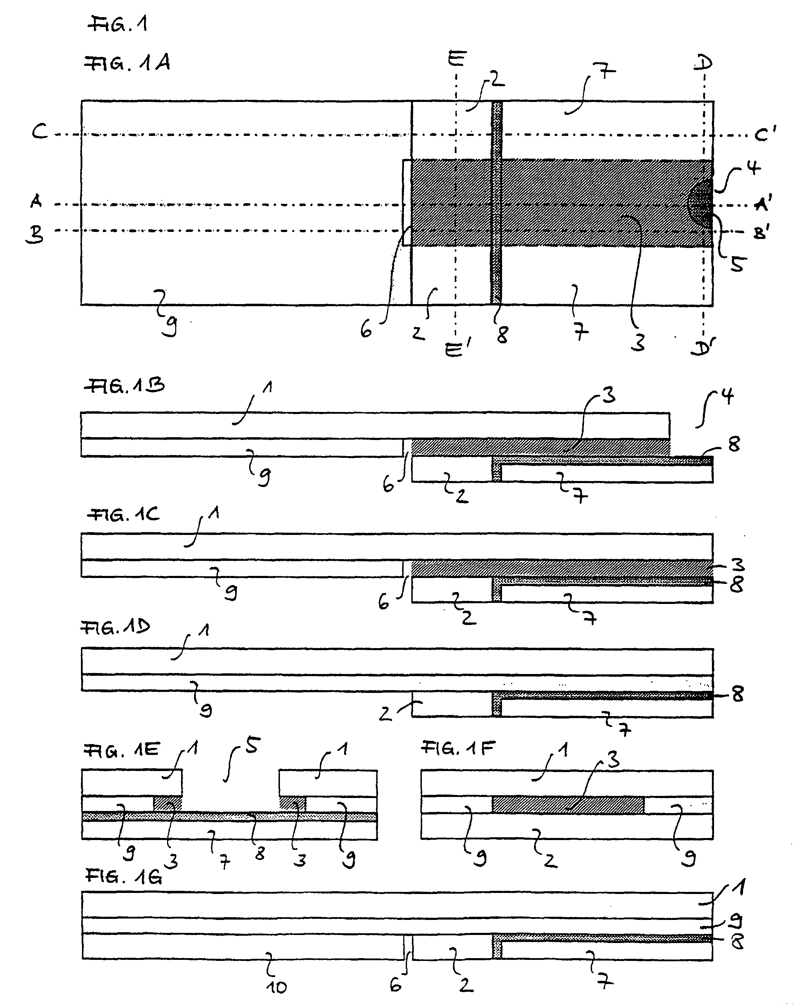

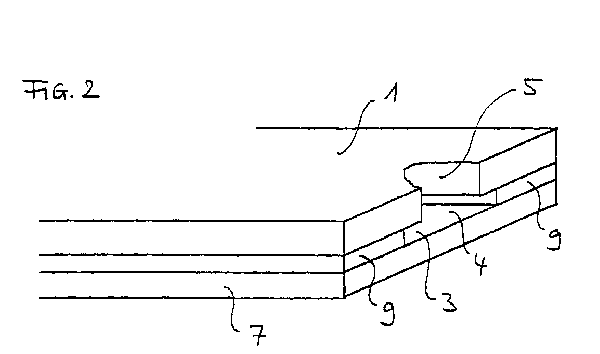

Die Erfindung wird durch die Figuren 1 und 2 und die nachfolgenden Beispiele näher erläutert.

Figur 1 zeigt eine besonders bevorzugte Ausführungsform der erfindungsgemäßen Vorrichtung. In Figur 1A ist eine schematische Aufsicht auf die erfindungsgemäße Vorrichtung abgebildet. Die Figuren 1B bis 1G zeigen jeweils Querschnitte entlang der Linien A-A' (1B), B-B' (1C), C-C' (1D und 1G), D-D' (1E) bzw. E-E' (1F).

Figur 2 zeigt eine perspektivische Detailvergrößerung des Probenaufgabebereichs der erfindungsgemäßen Vorrichtung.

Die Ziffern in den Figuren bedeuten:
- 1: Träger
- 2: Nachweiselement
- 3: Kapillarer Kanal
- 4: Probenaufgabeöffnung
- 5: Aussparung für Probenaufgabe
- 6: Entlüftungsöffnung
- 7: Abdeckung
- 8: Spaltabdeckfolie
- 9: Zwischenschicht
- 10: Stützfolie

In Figur 1 ist eine besonders bevorzugte Ausführungsform der erfindungsgemäßen Vorrichtung schematisch in verschiedenen Ansichten dargestellt (Figur 1A bis 1F). Die gezeigten Ansichten sollen einen plastischen Eindruck der erfindungsgemäßen Vorrichtung liefern. Auf einem inerten Träger (1) aus Kunststoff ist eine Zwischenschicht (9), beispielsweise in Form eines doppelseitig klebenden Bandes, angebracht. Die Zwischenschicht (9) besitzt im Bereich des kapillaren Kanals (3) eine Aussparung, die Länge und Breite des Kanals (3) bestimmt. Seine Höhe ist durch die Dicke der Zwischenschicht (9) vorgegeben. Auf der dem Träger (1) gegenüberliegenden Seite des Kapillarkanals (3) befindet sich neben dem Nachweiselement (2), z.B. einer reagenzimprägnierten Membran, eine Abdeckung (7), beispielsweise eine Kunststoffolie. Um kapillaren Schluß zwischen Nachweiselement und Abdeckung zu gewährleisten ist eine Spaltabdeckfolie (8) vorgesehen. Diese kann hydrophiliert sein, so daß sie einen schnellen Probentransport von der Probenaufgabeöffnung (4) zur Entlüftungsöffnung (6) ermöglicht, die das entgegengesetzte Ende des kapillaren Kanals markiert. Die Hydrophilierung hat daneben den Vorteil, daß im Bereich der Aussparung (5) ein Probenflüssigkeitstropfen direkt auf eine hydrophile Fläche aufgebracht werden kann, die an mehreren Begrenzungsseiten von kapillaraktiver Zone (3) umgeben ist. Dies führt zu einem raschen Eindringen des Flüssigkeitstropfens in das Testelement.

Die Kapillarzone (3) reicht von der Probenaufgabeöffnung (4) bis zum entgegengesetzten Ende des Nachweiselements (2) und gewährleistet somit einen vollflächigen Kontakt des Nachweiselements (2) mit Probenflüssigkeit und damit eine homogene Probenverteilung über das Nachweiselement (2). Probenaufgabeöffnung (4) und Entlüftungsöffnung (6) begrenzen den kapillaraktiven Bereich (3) in Richtung des kapillaren Transports.

In Figur 1 G ist gezeigt, wie die Zwischenschicht (9) durch eine Stützfolie (10) abgedeckt werden kann, um freiliegende Klebebandbereiche zu bedecken. Die Entlüftungsöffnung (6) darf dabei jedoch nicht überdeckt werden.

Bei der Verwendung der gezeigten Vorrichtung wird diese mit der Probenaufgabeöffnung (4) beispielsweise an einen sich an der Fingerkuppe befindlichen Blutstropfen gebracht. Dabei spielt es keine Rolle, ob der Blutstropfen von oben, das heißt von der flachen Seite des Trägers (1), oder von vorne, das heißt von der Stirnseite des Testelements, die die Probenaufgabeöffnung (4) enthält, mit der erfindungsgemäßen Vorrichtung kontaktiert wird. Ein fehlerhafter Probenauftrag, wie er beispielsweise bei Benutzern zu erwarten ist, die herkömmliche, "von oben" zu dosierende Teststreifen gewöhnt sind, ist somit weitgehend ausgeschlossen. Bei der Verwendung der erfindungsgemäßen Vorrichtung kommt der Blutstropfen durch die Aussparung (5) im Träger (1) mit der freiliegenden Fläche, die gegebenenfalls hydrophiliert ist, und gleichzeitig dem kapillaren Kanal (3) in Kontakt. Letzterer füllt sich solange mit Probe, bis er von der Probenaufgabeöffnung (4) bis zur Entlüftungsöffnung (6) gefüllt ist. Danach wird die Vorrichtung vom Patientenfinger entfernt, wodurch gewährleistet ist, daß lediglich die sich im Kapillarkanal (3) befindliche Probe für das Nachweiselement (2) verfügbar ist. Eine Überdosierung wird damit ausgeschlossen. Die definierte Höhe des kapillaraktiven Kanals sorgt für eine definierte Schichtdicke der Probe am Nachweiselement.

In Figur 2 ist eine Detailvergrößerung in perspektivischer Ansicht des Probenaufgabebereiches einer besonders bevorzugten Ausführungsform der erfindungsgemäßen Vorrichtung abgebildet. Die Aussparung (5) im Träger (1) erleichtert das Eindringen einer Probenflüssigkeit von der Probenaufgabeöffnung (4) in die kapillaraktive Zone (3), die im vorliegenden Fall von Träger (1), Zwischenschicht (9) und Abdeckung (7) gebildet wird. Die Aussparung kann neben der gezeigten Form auch jede andere, beliebige Form besitzen, die dem erfindungsgemäßen Zweck dienlich ist. Unter anderem sind halbrunde, drei- oder vieleckige Formen und die Verwendung einer oder mehrerer, nebeneinanderliegender oder versetzt gegenüberliegender Aussparungen möglich.

### Beispiel 1

### Herstellung des erfindungsgemäßen analytischen Testelements

Auf eine mit einer 30 nm dicken Schicht aus Aluminium, das mit Wasserdampf vollständig oxidiert wurde, beschichtete, 350 µm dicke Folie aus Polyethylenterephthalat (Melinex®, ICI, Frankfurt am Main, Deutschland) wird ein doppelseitiges Klebeband der Dicke 100 µm geklebt. Die Folie hat eine Länge von 25 mm und ist 5 mm breit. An einer der kurzen Seiten befindet sich eine zentrale, dreieckig-keilförmige Aussparung von 1 mm Breite und 2 mm Länge. Das Klebeband besitzt eine Ausstanzung von 2 mm Breite und mehr als 15 mm Länge, die die Dimensionen des Kapillarkanals definieren. Die Länge der Ausstanzung ist geringfügig größer zu wählen als die gewünschte Länge des kapillaraktiven Kanals, die durch dessen Abdeckung bestimmt wird, um eine Entlüftung des Kanals während des Befüllens mit Probenflüssigkeit zu gewährleisten. Auf das Klebeband wird auf der Seite, an der die Entlüftung vorgesehen ist, in 1 mm Abstand vom Ende der Ausstanzung ein 3 mm langer und 5 mm breiter Nachweisfilm geklebt. Als Nachweisfilm wird ein Film verwendet, wie er aus der deutschen Patentanmeldung DE-A-196 29 656 bekannt ist. Der Nachweisfilm ist spezifisch für den Nachweis von Glucose. Auf den noch offen liegenden Bereich des Klebebandes zwischen kerbenförmiger Aussparung und Nachweisfilm wird eine 12 mm lange und 5 mm breite Abdeckschicht aufgeklebt, so daß Abdeckschicht und Nachweisfilm Stoß an Stoß zu liegen kommen. Die Abdeckschicht besteht aus einer 150 µm dicken, einseitig mit Klebstoff versehenen Polyethylenterephthalat-Folie, auf die auf der zum Kapillarkanal hingewandten Seite eine mit oxidiertem Aluminium der Dicke 30 nm beschichtete, 6 µm dicke

Polyethylenterephthalat-Folie geklebt ist ( beide: Hostaphan®, Hoechst, Frankfurt am Main, Deutschland). Die dünnere Folie steht dabei an der zum Nachweisfilm gewandten Seite ca. 500 µm über die dickere Folie über. Bei der Montage der Abdeckschicht auf das Klebeband ist darauf zu achten, daß das überstehende Ende der dünneren Folie zwischen dem Nachweiselement und der dickeren Folie der Abdeckschicht zu liegen kommt. Um noch frei liegende Klebebandbereiche abzudecken werden diese mit einer 175 µm dicken Melinex®-Folie abgedeckt, ohne dabei jedoch funktionale Bereiche abzudecken.

Das so erhaltene Testelement hat eine kapillaren Kanal von 15 mm Länge, 2 mm Breite und 0,1 mm Höhe. Der Kanal kann 3 µl Probenflüssigkeit aufnehmen. Der Nachweisfilm wird auf einer Fläche von 3 mm x 2 mm von der Probe benetzt.

### Beispiel 2

### Messung der Blutglukosekonzentration mit Hilfe des Testelements aus Beispiel 1

Das Testelement aus Beispiel 1 wird mit der Probenaufgabeseite auf einen Probenflüssigkeitstropfen aufgesetzt. Die Kapillare des Testelements füllt sich innerhalb von 2 s selbständig mit Probe. Ist Glucose in der Probe vorhanden wird nach wenigen Sekunden eine Farbentwicklung im Nachweisfilm sichtbar. Nach ca. 30 bis 35 s ist der Endpunkt der Reaktion erreicht. Die erhaltene Farbe kann mit der Glucosekonzentration der Probe korreliert werden und wird entweder visuell oder reflexionsfotometrisch ausgewertet.

## Patentansprüche

1. Vorrichtung zur Probennahme von flüssigen Proben für analytische Elemente, bei der die Probe in einem kapillaraktiven Kanal (3) vom Probennahmeort zum Bestimmungsort transportiert wird und bei der der kapillaraktive Kanal (3) im wesentlichen von einem Träger (1), einer Abdeckung (7) und gegebenenfalls einer zwischen Abdeckung (7) und Träger (1) liegenden Zwischenschicht (9) gebildet wird, **dadurch gekennzeichnet, daß** sich eine Aussparung (5) in Form eines teilweisen Einschnitts in einer den zum kapillaren Flüssigkeitstransport befähigten Kanal (3) bildenden Fläche an der die Probenaufgabeöffnung (4) enthaltenden Seitenfläche der Vorrichtung befindet, wobei der Einschnitt an der Seitenfläche der Vorrichtung beginnt und vom Probennahmeort in Richtung des kapillaraktiven Kanals (3) weist, so daß die der Aussparung (5) gegenüberliegende Fläche frei liegt.

2. Vorrichtung gemäß Anspruch 1, **dadurch gekennzeichnet, daß** sich mindestens zwei Aussparungen nebeneinander befinden.

3. Vorrichtung gemäß Anspruch 1, **dadurch gekennzeichnet, daß** sich mindestens zwei Aussparungen versetzt auf gegenüberliegenden Seiten befinden.

4. Vorrichtung gemäß einem der Ansprüche 1 bis 3, **dadurch gekennzeichnet, daß** zumindest eine der die innere Oberfläche des zum kapillaren Flüssigkeitstransports befähigten Kanals bildenden Flächen hydrophiliert ist.

5. Vorrichtung gemäß Anspruch 4, **dadurch gekennzeichnet, daß** die der Aussparung gegenüberliegende Fläche hydrophiliert ist.

6. Vorrichtung gemäß Anspruch 4 oder 5, **dadurch gekennzeichnet, daß** die Hydrophilierung durch die Verwendung eines hydrophilen Materials oder durch Beschichtung eines wenig hydrophilen Materials mit einer hydrophilen Schicht erreicht wird.

7. Vorrichtung gemäß Anspruch 6, **dadurch gekennzeichnet, daß** zur Hydrophilierung eine Schicht aus oxidiertem Aluminium verwendet wird.

8. Verfahren zur Aufnahme einer flüssigen Probe in ein analytisches Element mit Hilfe einer Vorrichtung gemäß einem der Ansprüche 1 bis 7, wobei die flüssige Probe an der durch die Aussparung unterbrochenen Seitenfläche der Vorrichtung mit dieser kontaktiert wird und durch Kapillarkräfte in den zum kapillaren Flüssigkeitstransport befähigten Kanal transportiert wird.

## Claims

1. Device for withdrawing samples of liquid samples for analytical elements in which the sample is transported in a capillary-active channel (3) from the sampling site to the determination site and in which the capillary-active channel (3) is essentially formed by a carrier (1), a cover (7) and optionally an intermediate layer (9) situated between the cover (7) and carrier (1), wherein a notch (5) in the form of a partial recess is located in one of the surfaces forming the channel capable of capillary liquid transport (3) on the lateral face of the device containing the sample application opening (4), the notch beginning at the lateral face of the device and pointing from the sampling site towards the capillary-active channel (3) in such a manner that the surface opposite to the notch (5) is exposed.

2. Device as claimed in claim 1, wherein at least two notches are located next to one another.

3. Device as claimed in claim 1, wherein at least two notches are staggered on opposite sides.

4. Device as claimed in one of the claims 1 to 3, wherein at least one of the surfaces forming the inner surface of the channel capable of capillary liquid transport is hydrophilized.

5. Device as claimed in claim 4, wherein the surface opposite to the notch is hydrophilized.

6. Device as claimed in claim 4 or 5, wherein the hydrophilization is achieved by use of a hydrophilic material or by coating a less hydrophilic material with a hydropholic layer.

7. Device as claimed in claim 6, wherein a layer of oxidized aluminium is used for the hydrophilization.

8. Process for withdrawing a liquid sample into an analytical element with the aid of a device as claimed in one of the claims 1 to 7, wherein the liquid sample is contacted with the lateral face of the device broken by the notch and is transported by capillary forces into the channel capable of capillary liquid transport.

## Revendications

1. Dispositif pour l'échantillonnage d'échantillons liquides pour des éléments analytiques, dans lequel l'échantillon est transporté dans un canal à activité capillaire (3) depuis l'endroit d'échantillonnage jusqu'à l'endroit de détermination, et dans lequel le canal à activité capillaire (3) est formé essentiellement par un support (1), par un recouvrement (7) et, le cas échéant, par une couche intermédiaire (9) disposée entre le recouvrement (7) et le support (1), **caractérisé en ce qu'**un évidement (5) sous la forme d'une encoche partielle est disposé dans une surface formant le canal (3) apte au transport capillaire de liquide contre la surface latérale du dispositif contenant l'ouverture (4) d'application de l'échantillon, l'encoche commençant contre la surface latérale du dispositif et étant orientée, à partir de l'endroit d'échantillonnage, dans la direction du canal à activité capillaire (3) de telle sorte que la surface opposée à l'évidement (5) est mise à nu.

2. Dispositif selon la revendication 1, **caractérisé en ce qu'**au moins deux évidements sont disposés l'un à côté de l'autre.

3. Dispositif selon la revendication 1, **caractérisé en ce qu'**au moins deux des évidements sont disposés à l'état décalé sur des côtés opposés.

4. Dispositif selon l'une quelconque des revendications 1 à 3, **caractérisé en ce qu'**au moins une des surfaces formant la surface interne du canal apte au transport capillaire de liquide a été rendue hydrophile.

5. Dispositif selon la revendication 4, **caractérisé en ce que** la surface opposée à l'évidement a été rendue hydrophile.

6. Dispositif selon la revendication 4 ou 5, **caractérisé en ce qu'**on obtient le caractère hydrophile en utilisant une matière hydrophile ou par enduction d'une couche hydrophile sur une matière peu hydrophile.

7. Dispositif selon la revendication 6, **caractérisé en ce qu'**on utilise, pour obtenir le caractère hydrophile, une couche constituée d'aluminium oxydé.

8. Procédé pour la réception d'un échantillon liquide dans un élément analytique à l'aide d'un dispositif selon l'une quelconque des revendications 1 à 7, dans lequel on met l'échantillon liquide en contact avec le dispositif contre la surface latérale de ce dernier interrompue par l'évidement et on le transporte via des forces capillaires dans le canal apte au transport capillaire de liquide.
